# EUROPEAN PATENT SPECIFICATION

(11) **EP 0 501 993 B1**
(45) Date of publication and mention of the grant of the patent: **11.06.1997**
(21) Application number: 90916676.1
(22) Date of filing: 21.11.1990
(51) Int. Cl.: G06T 17/00, A61B 5/11

(54) **PROBE-CORRELATED VIEWING OF ANATOMICAL IMAGE DATA**
SONDENKORRELIERTE ANZEIGE VON ANATOMISCHEN BILDDATEN
VISUALISATION DE DONNEES D'IMAGES ANATOMIQUES OBTENUES EN CORRELATION AVEC UNE SONDE

(30) Priority: 21.11.1989 CA 3497
(43) Date of publication of application: 09.09.1992
(73) Proprietor: I.S.G. TECHNOLOGIES INC., Ontario L4V 1S8 (CA)
(72) Inventor: GREENBERG, Michael, M., Richmond Hill, Ontario L4B 1R5 (CA); DEKEL, Doron, Willowdale, Ontario M5R 2R3 (CA); ZINREICH, Simion, J., Owings Mills, MD 21217 (US); BRYAN, Robert, N., Baltimore, MD 21200 (US)
(74) Representative: Skuhra, Udo, Dipl.-Ing.
(86) International application number: CA9000404
(87) International publication number: WO9107726

(56) References cited:
- WO-A-90/05494
- US-A- 4 638 798

## Description

### FIELD OF THE INVENTION

The invention relates to a method and a system for visualizing internal regions of an anatomical body according to the preambles of claims 1 and 20. More specifically, the invention relates to a method and a system for determining the position of a probe relative to various anatomical features and displaying the internal anatomical structures corresponding to the position of the probe.

### BACKGROUND OF THE INVENTION

In the document WO 88/09151, a process and device for the optical representation of surgical operations is described, whereat a coordinate measuring device is provided for detecting the position of the surgical instrument. Tomograms provided with at least three measurement points on the patient are stored in a computer and the position of three measurement points and that of the surgical instrument are determined. These positions are superimposed on corresponding tomographs on the screen. The purpose is the documenting of the performance and outcome of a surgical operation, in hidden parts of the body, the instantaneous position of the surgical position or its course are represented during the operation and recorded for subsequent examination. To this end, tomograms provided with at least three measurement points on the patient are stored in a computer and can be displayed on a screen. The described process and device are used in the representation and documentation of surgical operations.

From the US Patent No. 4,638,798, a stereotactic method and apparatus for treating a region of a patient's body is known defining points in the region using a three-dimensional coordinate system with reference to a ring attached to the patient for establishing a reference point for the three-dimensional coordinate system at the center of the ring. The ring and the reference point are used for stereotactically controlling instruments used to treat the region. The ring is provided with pins extending parallel to the axis of the ring, and equidistant from the center, for precise location of the center and of a base-line scan for correlation between the location of the region to be treated and the control system for treatment of the region. Prior to treatment, a series of no-ninvasive tomography scans are made through the region and at least part of the pins for determining the coordinate of at least one point of the region selected for the treatment with respect to the center of the ring. All parameters of this described system for stereotactic control of the instruments used for treatment are determined with respect to the ring center as a reference point.

In the WO 90/05494, a process and apparatus particularly suited for guiding neurosurgical operations is described. The described process for guiding neurosurgical operations allows a considerable reducton of surgical traumatism during the removal of cerebral lesions by virtue of the possibility of converting into three-dimensional images both the contours of the anatomical structures and the representation of the device for the stereotactic detection of the affected region together with a stereotactic probe which defines a surgical path. The apparatus requires a stereotactic detection device being a stereotactic helmet fitted on a patient.

In recent years, it has become commonplace for a surgeon to utilize slice images of a patient's internal organs. The images are used to plan the course of a medical procedure, be it diagnostic, therapeutic, or surgical, and for orientation during the procedure. The slice images are typically generated by Computerized Tomography (CT) or by Magnetic Resonance Imaging (MRI). Images may also be captured using Angiography, Single-Photon Emission Computed Tomography, and Positron Emission Tomography methods.

The images typically presented to a user consist of a series of static images on film. These images are very detailed and can resolve anatomical structures less than one millimetre in size. However, their format differs greatly from the actual anatomical features seen during the surgical procedure. The images are presented in two-dimensional form rather than in the three-dimensional form of the anatomical features. In addition, the perspective of the slice image rarely corersponds to the surgeon's viewing angle during the procedure. Consequently, during a procedure, the slice images provide a primitive visualization aid to the patient's anatomy.

To obtain proper orientation within a patient's body, surgeons can make an incision which is larger than the minimum required for the planned procedure. While providing an enlarged window to the patient's anatomy, these larger incisions may result in longer hospital stays and increased risk for the patient. On the other hand, if only a small incision is made, the field of view available to the surgeon is greatly limited. As a result, the surgeon may become disoriented forcing him to correct and recommence the procedure, or to continue at a high risk to the patient.

While imaging equipment can be used to provide on-the-spot visualization of a patient, it is impractical to use the equipment in the operating room during the procedure. First, the costs of purchasing, operating and maintaining the imaging equipment are prohibitive. Secondly, surgeons have limited access to a patient who is placed in a scanning device. Furthermore, Magnetic Resonance Imaging and Computerized Tomography have side effects which may harm the patient and inhibit the procedures. Magnetic Resonance Imaging produces a very high fixed magnetic field which precludes the use of many instruments. Computerized Tomography, on the other hand, utilizes X-ray radiation which is known to damage human tissue and cause cancer. It is, therefore, not desirable to expose a patient to a computerized tomography scan for a prolonged period.

A known approach to localizing anatomy during surgery is currently being used for brain lesions. The method is known as stereotactic surgery. It involves rigidly attaching the reference frame to the patient's head during the scanning. Using the marks left in the scanned images by the frame, the location of the lesion is computed. During the surgical procedure, a reference frame is again attached to the same location on the patient's head. The frame is used to direct drilling and cutting operations, which are done either manually or automatically.

Stereotactic surgery has a number of drawbacks. Firstly, it is only suitable for localized brain lesions which have a direct approach path. Secondly, stereotactic surgery requires the use of a cumbersome and uncomfortable reference frame. Furthermore, since the decision to undertake stereotactic surgery is usually done after a first scanning procedure, the patient must undergo a second scan with the frame attached. This results in a prolonged and expensive procedure. Moreover, if the scan utilizes computerized tomography imaging, then the patient is exposed to another dose of radiation.

Known in the art are systems and methods designed to allow the use of previously acquired Computer Tomography or Magnetic Resonance Imaging scans as an aide in conventional open neurosurgery. In general, these methods use systems comprising:
(a)a multi-jointed probe or sensor arm;
(b)a computer processing unit which calculates the position of the probe arm relative to certain reference points on the patent; and
(c)a means of displaying the superpositioning of the location of the probe arm as calculated above on the previously acquired scan images.

The display capabilities of such systems are limited in that they can display only the slice images as generated by the computerized tomography or magnetic resonance imaging scan. An example of such a system is disclosed in an international Application filed by Georg Schlöndovff and published under No. WO88/09151. This application is mainly concerned with an arm structure for locating the position of a probe.

### SUMMARY OF THE INVENTION

In a first embodiment of the invention provides a method for visualizing internal regions of an anatomical body in relation to a probe, employing a data-base body of previously acquired images of the anatomical body, the method comprising the steps of:
(a)obtaining a spatial position for the probe relative to the anatomical body;
(b)determining a data-base location relative to the data-base body corresponding to the spatial position of the probe relative to the anatomical body;
(c) mapping and registering the spatial position of the probe relative to the anatomical body to the corresponding data-base location of the probe relative to the data-base body; and
(d) displaying a region of the data-base body adjacent the data-base location of the probe, the region being derived from a plurality of adjacent images of the data-base body, characterized by sensing movement of each of said probe and said anatomical body to permit said obtaining of said spatial position for the probe relative to the anatomical body such as to permit the probe and the anatomical body to be independently displaced and such that registration between the data-base body and the anatomical body is maintained.

In a second aspect, the invention provides a system for visualizing internal regions of an anatomical body by utilizing a data-base body of previously acquired images of the anatomical body, the system comprising:
(a) a probe;
(b) a data-base storage unit containing the previously acquired images of the anatomical body;
(c) a spatial determinator for determining the spatial position of the probe relative to the anatomical body;
(d) a computer using the previously acquired images to generate a representation of a region of the anatomical body adjacent to the spatial position of the probe; and
(e) means to map and to register said spatial position of the probe relative to the anatomical body to the corresponding data-base location of the probe relative to the data-base body; and a display unit for displaying the representation of the anatomical body, characterized in that said probe and said anatomical body each has means to permit said determinator to determine the spatial position of the probe relative to the anatomical body such as to permit the probe and the anatomical body to be independently displaced such that registration between the data-base body and the anatomical body is maintained.

### BRIEF DESCRIPTION OF THE DRAWINGS

For a better understanding of the present invention, and to show more clearly how it may be carried into effect, reference will now be made by way of example to the accompanying drawings which show alternate embodiments of the present invention, and in which:
Figure 1 is a first embodiment of a probe-correlated imaging system;
Figure 2 is a portion of a known probe-correlated imaging system;
Figure 3 is a portion of a second embodiment of a probe-correlated imaging system;
Figure 4is a first display format employed in the system of fig. 1;
Figure 5is a second display format employed in the system of fig. 1; and
Figure 6is a third display format employed in the system of fig. 1.

### DETAILED DESCRIPTION OF THE PREFERRED EMBODIMENT

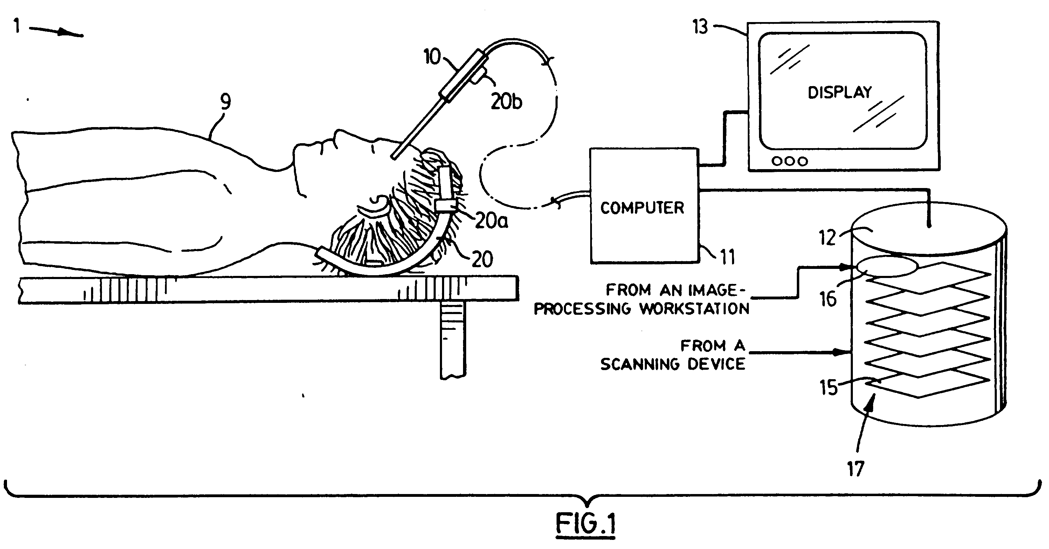

Referring to fig. 1 a probe-correlated system (1) has a probe (10), a computer (11), a data storage unit (12), and a display (13). These components are, individually, well known and common. The system (1) is employed to view the anatomical structure of a patient (9) adjacent to the position of the probe (10).

The computer (11) has ready access to the unit (12) which contains a data-base body (17) representing the anatomical structure of the patient (9). The data-base body (17) includes previously acquired digital images (15) of the patient (9). These images (15) can be acquired through various medical-imaging techniques, such as Computerized Tomography, Single-Photon Emission Computed Tomography, Positron Emission Tomography, Magnetic Resonance Imaging, Ultrasound, or Angiography.

In addition to the digital images (15) captured by medical-imaging techniques, the data-base body (17) can contain pre-processed digital images (16). For example, the digital images (15) together with their relative spatial relationship can be pre-processed to represent the various organ surfaces of the patient (9). There are known systems, not shown, which can read digital images (15) and generate pre-processed digital images (16) according to their relative spatial relationship within the anatomical structure of the patient (9). The known system places the pre-processed images (16) in the data-base body (17). The probe (10), or any other object which may function as a probe, is used by an operator, not shown, to point to a particular location on the anatomical body of the patient (9). The operator can move the probe (10) around or within the anatomical body of the patient (9).

Spatial coordinates, representing the spatial position and possibly the spatial orientation, of the probe relative to a fixed reference point, shown generally at the arrow (20), are conveyed to the computer (11). The reference point (20) may either be on the patient (9) as shown, or on some stable platform nearby, not shown. There are a number of alternate methods which can be used to obtain the spatial coordinates of the probe (10) relative to its reference point (20). The apparatuses described in association with such method will be collectively referred to as spatial determinators.

Referring to fig. 1, an electro-magnetic emitter (20a) is positioned at the reference point (20) and a sensor (20b) is located on the probe (10). By comparing the timing and phase of transmitted signals from the emitter (20a) to received signals picked up by the sensor (20b), the position and orientation of the probe (10) relative to the reference point (20) can be determined. A probe (10) using this known locating method is commercially available. Given the spatial relationship between the reference point (20) and the patient (9), the computer (11) can determine the position of the probe (10) relative to the patient (9).

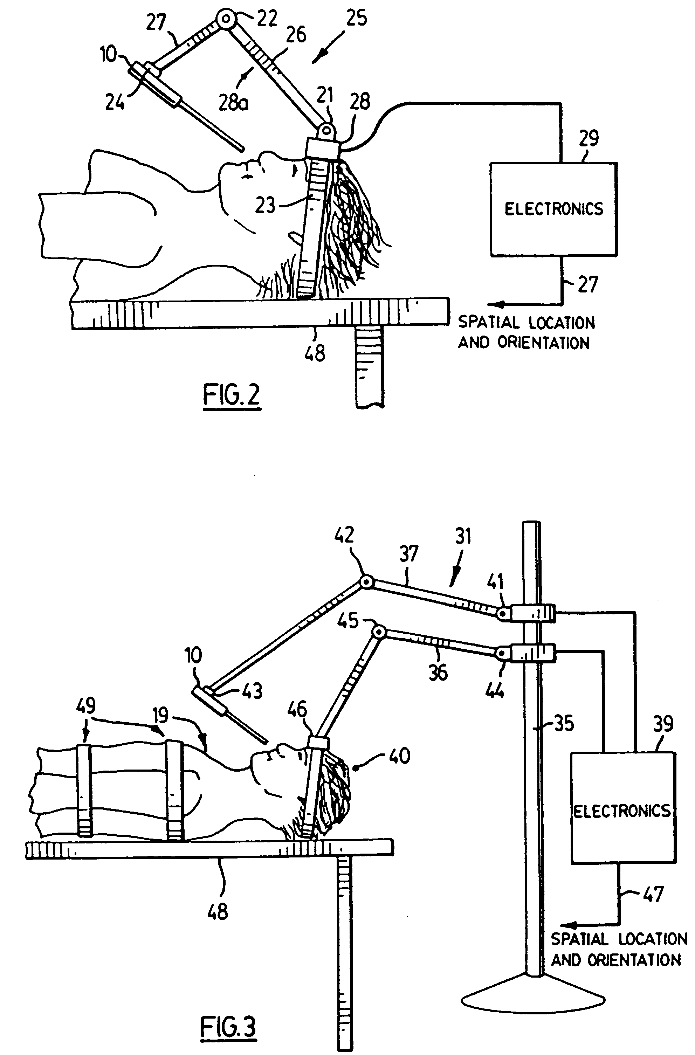

Referring to fig. 2, it is known to attach the probe (10) to a multi-joint light-weight arm (25) with a first section (26) and a second section (27) connected together at joint (22). The first section (26) of the multi-joint arm (25) is connected to a base (28) at joint (21). The base (28) is attached to the patient (9) using adhesive elastic tape (23). The probe (10) is attached to the second section (27) at joint (24).

The joints (21), (22), (24), in combination, provide for a range of motion equal to or greater than that required for a given procedure. Angular sensors, not shown, are located at the joints (21), (22), (24).

The angular sensors are connected by wire (28a) to one another and to an electronic unit (29). The sensors detect any change in the position or orientation of the multi-joint arm (25), and convey this information to the electronic unit (29).

The unit (29) uses geometric calculations to determine the spatial position and spatial orientation of the probe (10) relative to the base (28) which is used as the reference point. The spatial position and spatial orientation of the probe (10) are sent to the computer (11) of fig. 1 through an electronic communication link (27). A suitable communication link (27) would be an RS-232 serial communication interface. Since the base (28) is fixed to the body of the patient (9), the computer can use the spatial information to determine the position of the probe (10) relative to the patient (9).

Alternately, referring to fig. 3, in a second embodiment a dual-arm arrangement, shown generally at (31), may be employed. The arrangement (31) is particularly effective where the multi-joint arm (30) of fig. 2 cannot be fixed to the patient (9).

A stand (35) is used to anchor two multi-joint arms (36, 37) similar to the multi-joint arm (30) of fig. 2. A probe (10) is attached to the other end of arm (37). Arm (36) is attached at its other end to a reference point (40) on the patient (9). Sensors are mounted at joints (41, 42, 43) of arm (37), and at joints (44, 45,46) of arm (36). The sensors, in turn, are connected to an electronic unit (39). The electronic unit (39) decodes the position and orientation of the probe (10). Through the relative spatial positions and orientations of the probe (10) to the joint (41), the joint (41) to the joint (44) and the joint (44) to the reference point (40), the spatial position and orientation of the probe (10) relative to the patient (9) is obtained. The spatial position and orientation of the probe (10) is transmitted to the computer (11) of fig. 1 via the communication link (47).

The reference arm (36) shown in fig. 3 can be omitted if the patient (9) is fixed to an operating table (48). The patient can be fixed to the table (48) using straps (49). If the patient (9) is fixed, then the reference point (40) can be fixed arbitrarily in space. The relative position of the reference point (40) to the joint (41) may be determined once and the relative position of the probe (10) to the reference point (40) determined therefrom. However, if the patient (9) is moved during the procedure, a new reference point (40) or a new spatial relationship must be established.

To display the data-base image (15) or pre-processed image (16) which correctly corresponds to the region of the anatomical body of the patient (9) adjacent the probe (10), the system (1) must be able to map positions of the anatomical body of the patient (9) to locations in data-base body (17) during the procedure. In this sense mapping is a procedure for determining the current spatial position of the probe (10) and the corresponding adjacent data-base body (17) location. This correspondence may be initially determined through a procedure which maps the patient (9) to the data-base body (17). This procedure is known as "registration" since its purpose is to register the correspondence between the anatomical body of the patient (9) and the data-base body (17) with the computer (11).

A number of registration methods are known. For example, one method involves the marking of reference points on the patent (9). However, this can be inconvenient and there is a risk that the marked positions on the patient (9) may be erased between the time the scan images (15) were generated and the time the surgical procedure is performed. Another method involves placing small markers, usually made of cad or ceramic material, on readily identifiable features of the patent, such as the ears or the corners of the eyes.

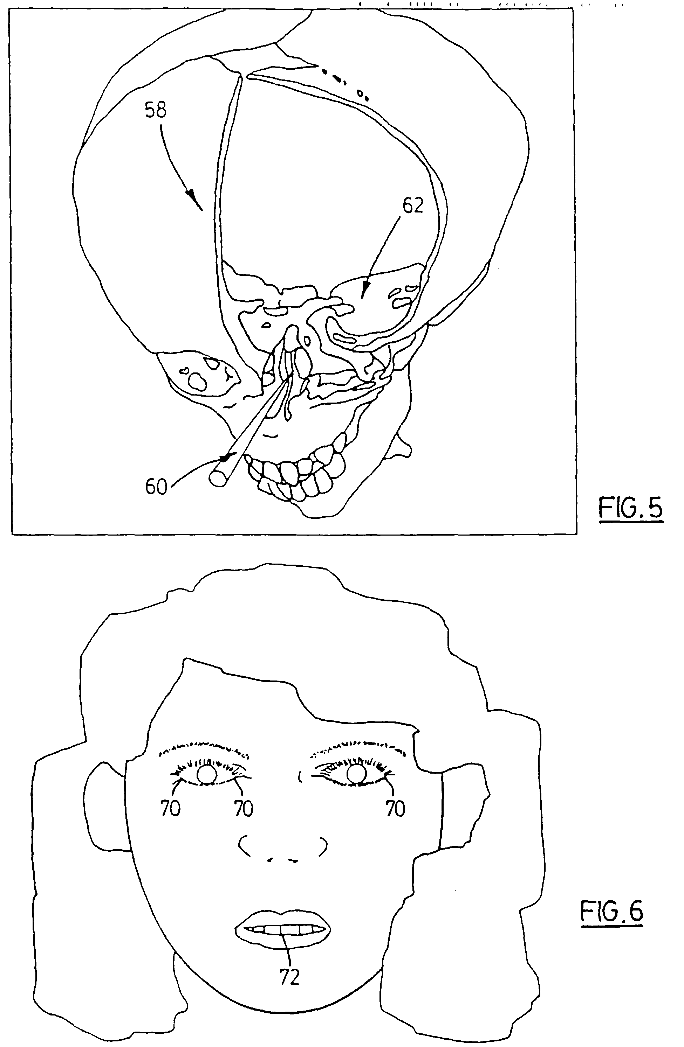

The preferred registration method involves using the probe (10) to register with the computer (11) the spatial position of easily identifiable features of the patient, such as the space between the teeth, the nose or the corners of the eyes. In this method, the previously acquired scan images (15) or the pre-processed images (16) are displayed on the display (13) in such a manner as to allow the user of the system (1) to identify specific points of the chosen features of the patient (9). A three dimensional surface format, shown in figure 6, is the simplest such format for an unskilled viewer to comprehend. Such a three-dimensional surface format can be derived from the pre-processed images (16) in a known manner, and suitable points such as the corners of the eyes (70), space between the teeth (72) are shown in figure 6.

The method is as follows. The probe (10) is placed next to the feature point on the patient (9). The spatial position of the probe (10) is then determined. A movable marker, e.g. a cursor, on the display (13) is then adjusted so it coincides with a selected feature, e.g. corner of the eyes (70) as seen. It is then relatively simple for the computer (11) to perform necessary three dimensional transformation, so that the spatial position of the probe (10) and the corresponding data-base body location are registered with the computer (11). Using a set of at least three, and preferably about six, feature points on the patient, a proper and unique transformation function, can be calculated which maps the spatial position of the probe (10) to the corresponding data-base body location and orientation. The accuracy of this transformation function is improved by the use of a larger number of points and a statistical error minimizing techniques, such as the least mean square error method.

Once the anatomical body of the patient (9) has been registered with the computer (11), the operator can move the probe (10) in and around the patient (9), and at the same time view the hidden anatomical features of the patient (9) as they appear in the data-base body (17). The anatomical features of the patient (9) in the data-base body (17) are presented on the display unit (13) in relationship to the spatial position and possibly orientation of the probe (10).

It is not strictly necessary to use the orientation of the probe (10) to carry out many of the features of the invention. The probe (10) may be represented on the display (13) as a point rather than a full probe (10). The region adjacent the point probe (10) is then displayed. The orientation of the regions displayed is known from the computer (11) and not determined by the orientation of the probe (10).

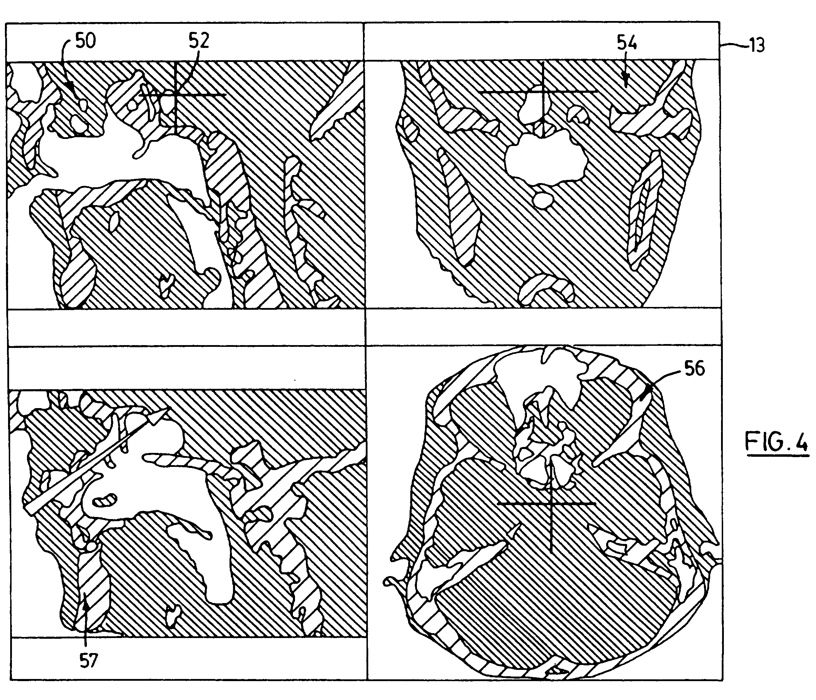

A possible presentation format for the data-base images (15) of the patient (9) is shown in fig. 4. Two-dimensional representations or slice images are generated by the computer (11) from the data-base images (15). The position of the probe (10) relative to the anatomical body (9) is marked on a slice image (50) by the computer (11). The slice image (50) together with the probe (52) are displayed on the unit (13).

The screen of the display unit (13) is divided into 4 separate windows. Three of the windows contain slice images corresponding to three cardinal anatomical planes: sagittal (50); axial (54); and coronal (56). The three slice images (50, 54, 56) intersect at the location of the probe (52). Thus, the operator can observe the anatomical feature of the patient (9) relative to the position of the probe (10) in the six main directions: anterior, posterior, superior, inferior, right and left. The fourth window depicted on the display unit (13) can show a slice (57) through the anatomical features in mid-sagittal orientation along the axis of the probe (10). The position and orientation of the probe (10) can be marked on the slice (57), thereby allowing the operator to direct viewing of what lies ahead of the probe (10).

Another presentation format for the data-base images (15) and pre-processed images (16) is shown in fig. 5. A three-dimensional model (58) of the patient (9) is generated by the computer (11) from the images (15, 16). The computer (11) also generates a three-dimensional model (60) of the probe (10). The relative locations of the models (60), (58) correspond to the spatial position and orientation of the probe (10) relative to the patient (9). The three-dimensional model (58) of the patient (9) generated from the stored images (15, 16) is presented together with the model (60) on the display unit (13).

Other display methods than the display of slices or using pre-processing may be used in conjunction with the principles described herein. For example, the computer (11) can generate displays directly from the images (15) using a ray-cast method. In one ray-cast method the computer (11) creates the display using the results of simulated X-rays passing through the images (15). The simulated X-rays will be affected differently by different elements in the images (15) according to their relative absorption of the X-rays. The results may be displayed along with the probe (10) in a manner similar to those described for slices or 3d-images. This produces a simulated X-ray display. In another ray-cast method a display is created using the results of simulated light rays passing through the images (15). The elements in the images (15) which do not pass the simulated light rays correspond to surface features and may be used to generate a display similar to the three-dimensional model (58).

There are other ray casting methods which are well known in the art.

The computer (11) can be used to further process the slice image (50) and three-dimensional images (58) generated from the data-base body (17). For example, a wedge-shaped portion (62) has been cut from the three-dimensional image (58). The cut-out portion (62) exposes various structures adjacent to the probe (10), which would not otherwise be observable. In addition, the cut-out portion (62) gives the operator an unobstructed view of the position of the probe (10) even if it is within the patient (9). The slice images (50) and three-dimensional images (58), (60) can also be processed by the computer (11) using other known image processing techniques. For example, the model (60) of the probe (10) can be made translucent, or the slice image (50) can be combined with other slice views.

## Claims

1. A method for visualizing internal regions of an anatomical body (9) in relation to a probe (10), employing a data-base body (17) of previously acquired images of the anatomical body (9), the method comprising the steps of:
(a) obtaining a spatial position for the probe (10) relative to the anatomical body (9);
(b) determining a data-base location relative to the data-base body (17) corresponding to the spatial position of the probe (10) relative to the anatomical body (9);
(c) mapping and registering the spatial position of the probe (10) relative to the anatomical body (9) to the corresponding data-base location of the probe (10) relative to the data-base body (17); and
(d) displaying a region of the data-base body (17) adjacent the data-base location of the probe (10), the region being derived from a plurality of adjacent images of the data-base body (17),
**characterized** by
the step of sensing movement of each of said probe (10) and said anatomical body (9) to permit said obtaining of said spatial position for the probe (10) relative to the anatomical body (9) such as to permit the probe (10) and the anatomical body (9) to be independently displaced and such that registration between the data-base body (17) and the anatomical body (9) is maintained.

2. The method according to claim 1,
**characterized** in that
displaying a region of the data-base body (17) adjacent to the data-base location of the probe (10) comprises the steps of:
(a) generating a slice image from the previously acquired images and intersecting a plurality of adjacent images, representing a region of the data-base body (17) adjacent to a data-base location of the probe (10); and
(b) displaying the slice image.

3. The method according to claim 1,
**characterized** in that
displaying a region of the data-base body (17) adjacent to the data-base location of the probe (10) comprises the steps of:
(a) generating a three-dimensional body model from the previously acquired images representing a region of the data-base body adjacent to the data-base location of the probe; and
(b) displaying the three-dimensional body model.

4. The method according to claim 1,
**characterized** in that
displaying a region of the data-base body adjacent to the data-base location of the probe comprises the steps of:
(a) generating a three-dimensional body model from previously acquired images which have been pre-processed to depict anatomical features and which represent a region of the data-base body adjacent
to the data-base location of the probe; and (b) displaying the three-dimensional body model.

5. The method according to claim 4,
**characterized** in that
in step (a) a portion of the three-dimensional body model is removed to reveal a location corresponding to the location of the probe (10).

6. The method according to claim 1,
**characterized** in that
displaying a region of the data-base body (17) adjacent to the data-base location of the probe (10) comprises the steps of:
(a) generating a display format through the use of a ray-cast method on previously acquired images representing a region of the data-base body adjacent to the data-base location of the probe; and
(b) displaying the display format.

7. The method according to claim 6,
**characterized** in that
the ray-cast method is selected from the group consisting of X-ray and light ray.

8. The method according to claim 6 or 7,
**characterized** in that
the display format is a three-dimensional format.

9. The method according to claim 1, 2 or 3,
**characterized** in that
the spatial orientation of the probe (10) is obtained along with its spatial position.

10. The method according to claim 1, 2 or 3,
**characterized** in that
a representation of the probe is displayed along with the region of the data-base body (17) adjacent the data-base location of the probe (10) and the relative locations of the representation of the probe (10) and the data-base body (17) correspond to the spatial position of the probe (10) relative to the anatomical body (9).

11. The method according to claim 5,
**characterized** in that
a representation of the probe (10) is displayed with the three-dimensional body model, the relative location of the representation of the probe (10) to the three-dimensional body model corresponding to the spatial position of the probe (10) relative to the anatomical body (9).

12. The method according to claim 11,
**characterized** in that
the representation of the probe (10) corresponds closely to the actual probe, wherein the representation of the probe is additionally oriented to correspond to the orientation of the probe with respect to the anatomical body (9), and with the perspective of the representation of the probe (10) and of the three-dimensional body model corresponding to one another.

13. The method according to claim 1,
**characterized** by
further comprising a step for registration prior to obtaining the spatial position, registration including the steps of:
(a) positioning the probe (10) next to a particular feature of the anatomical body (9);
(b) determining a spatial position for the probe (10);
(c) displaying a region of the data-base body (17) having a data-base body feature corresponding to the particular feature;
(d) identifying the particular feature on the displayed region; and
(e) registering the spatial position of the probe and the location on the data-base body corresponding to the position of the particular feature; whereby, a data-base location is determined to correspond with a spatial position of the probe (10).

14. The method according to claim 1,
**characterized** by
further comprising a step for registration prior to obtaining the spatial position, registration including the steps of:
(a) marking locations in the data-base body (17) which correspond to particular features of the anatomical body (9);
(b) positioning the probe (10) next to a particular feature of the anatomical body (9);
(c) determining the spatial position of the probe (10);
(d) registering the spatial position of the probe (10) and its corresponding data-base body location,
whereby, a data-base body location is determined to correspond with a spatial position of the probe (10).

15. The method according to claim 1,
**characterized** by
further comprising a step for registration prior to obtaining the spatial position, registration including the steps of:
(a) marking a position on the anatomical body (9) of a particular scanned image containing a corresponding location in the data-base body (17);
(b) positioning the probe (10) next to the marked position on the anatomical body (9);
(c) determining the spatial position of the probe (10);
(d) registering the spatial position of the probe (10) and its corresponding data-base body location,
whereby a data-base body location is determined to correspond with a spatial position of the probe (10).

16. The method according to claim 13,
**characterized** in that
the display of step (c) is a three-dimensional display.

17. The method according to claims 13 or 16,
**characterized** in that
more than three data-base locations are identified and taht errors between the corresponding data-base locations and spatial positions are minimized to improve the accuracy of the registration step.

18. The method according to claim 17,
**characterized** in that
the errors are minimized using at least mean squares analysis.

19. The method according to claim 13,
**characterized** in that
the probe (10) is connected to the anatomical body (9) in such a manner that, following registration, if the anatomical body (9) is displaced, registration between the data-base body (17) and the anatomical body (9) is maintained.

20. A system for visualizing internal regions of an anatomical body by utilizing a data-base body (17) of previously acquired images of the anatomical body (9), the system comprising
a probe (10);
a data-base storage unit (12) containing the previously acquired images of the anatomical body (9);
a spatial determinator (20a, 20b, 36, 37, 39) for determining the spatial position of the probe (10) relative to the anatomical body (9);
a computer (11) using the previously acquired images to generate a representation of a region of the anatomical body (9) adjacent to the spatial position of the probe (10);
means (11) to map and to register said spatial position of the probe relative to the anatomical body (9) to the corresponding data-base location of the probe (10) relative to the data-base body (9); and
a display unit (13) for displaying the representation of the anatomical body,
**characterized** in that
said probe (10) and said anatomical body (9) each has means (20a, 20b, 26, 27) to permit said determinator to determine the spatial position of the probe (10) relative to the anatomical body (9) such as to permit the probe (10) and the anatomical body (9) to be independently displaced such that registration between the data-base body (17) and the anatomical body (9) is maintained.

21. The system according to claim 20,
**characterized** in that
the generated representations are displayed in a three-dimensional surface format.

22. The system according to claim 20,
**characterized** in that
the computer (11) is adapted to be initialized for the location in the data-base storage unit corresponding to the spatial position of the probe (10) by having the probe positioned next to a particular feature point of the anatomical body (9), determining a spatial position of the probe (10), displaying a region of the data-base body (17) having a data-base body feature corresponding to the particular feature on the displayed region, and registering the spatial position of the probe (10) and the location on the data-base body (17) corresponding to the position of the particular feature.

23. The system according to claim 22,
**characterized** in that
the generated images are displaced in three-dimensional format during registration, and the particular features are identified on the three-dimensional format.

24. The system according to claim 20,
**characterized** in that
the spatial determinator (20a, 20b, 36, 37, 39) includes:
(a) an electro-magnetic emitter on a reference point for transmitting a signal;
(b) a sensor on the probe (10) for receiving the signal; and
(c) means for comparing the transmitted signal with the received signal to determine the position of the probe (10).

25. The system according to claim 20,
**characterized** in that
the spatial determinator (20a, 20b, 36, 37, 39) includes:
(a) first, second, third and fourth sections;
(b) a stand;
(c) a first joint between the first section and the probe;
(d) a second joint between the first and second sections;
(e) a third joint between the second section and the stand;
(f) a fourth joint between the stand and the third section;
(g) a fifth joint between the third section and the fourth section;
(h) a sixth joint between the fourth section and a reference point whose spatial position relative to the anatomical body is known;
(i) sensors positioned at each of the joints; and
(j) means connected to the sensors for determining the position of the probe relative to the anatomical body.

26. The system according to claim 20,
**characterized** in that
the spatial determinator (20a, 20b, 36, 37, 39) determines the spatial orientation of the probe (10) as well as its spatial position.

## Patentansprüche

1. Verfahren zum Sichtbarmachen innerer Bereiche eines anatomischen Körpers (9) in Bezug auf eine Sonde unter Verwendung eines Datenbankkörpers (17) zuvor aufgenommener Bilder des anatomischen Körpers (9), welches folgende Schritte aufweist:
a) Einrichten einer räumlichen Position für die Sonde (10) relativ zum anatomischen Körper (9);
b) Bestimmen eines Datenbankorts relativ zum Datenbankkörper (17) entsprechend der räumlichen Position der Sonde (10) relativ zum anatomischen Körper (9);
c) Abbilden und Registrieren der räumlichen Position der Sonde (10) relativ zum anatomischen Körper (9) auf dem entsprechenden Datenbankort der Sonde (10) relativ zum Datenbankkörper (17); und
d) Anzeigen eines Bereichs des Datenbankkörpers (17) neben dem Datenbankort der Sonde (10), wobei der Bereich von einer Vielzahl benachbarter Bilder des Datenbankkörpers (17) abgeleitet wird,
gekennzeichnet durch
den Schritt des Erfassens der Bewegung von sowohl der Sonde (10) als auch des anatomischen Körpers (9) zum Erhalten der räumlichen Position für die Sonde (10) relativ zum anatomischen Körper (9), derart daß die Sonde (10) und der anatomische Körper (9) unabhängig verschoben werden, und derart, daß die Registrierung zwischen dem Datenbankkörper (17) und dem anatomischen Körper (9) aufrechterhalten wird.

2. Verfahren nach Anspruch 1,
**dadurch gekennzeichnet,**
daß das Anzeigen eines Bereichs des Datenbankkörpers (17) neben dem Datenbankort der Sonde (10) folgende Schritte aufweist:
a) Erzeugen eines Schnittbildes aus den zuvor aufgenommenen Bildern und Schneiden einer Vielzahl der benachbarten Bilder, darstellend eines Bereichs des Datenbankkörpers (17) neben einem Datenbankort der Sonde (10); und
b) Anzeigen des Schnittbilds.

3. Verfahren nach Anspruch 1,
**dadurch gekennzeichnet,**
daß das Anzeigen eines Bereichs des Datenbankkörpers (17) neben dem Datenbankort der Sonde (10) folgende Schritte aufweist:
a) Erzeugen eines dreidimensionalen Körpermodells aus dem zuvor aufgenommenen Bildern, darstellend einen Bereich des Datenbankkörpers neben dem Datenbankort der Sonde; und
b) Anzeigen des dreidimensionalen Körpermodells.

4. Verfahren nach Anspruch 1,
**dadurch gekennzeichnet,**
daß das Anzeigen eines Bereichs des Datenbankkörpers neben dem Datenbankort der Sonde folgende Schritte aufweist:
a) Erzeugen eines dreidimensionalen Körpermodells aus zuvor aufgenommenen Bildern, welche zur Abbildung anatomischer Merkmale vorverarbeitet sind und welche einen Bereich des Datenbankkörpers neben dem Datenbankort der Sonde darstellen; und
b) Anzeigen des dreidimensionalen Körpermodells.

5. Verfahren nach Anspruch 4,
**dadurch gekennzeichnet,**
daß in Schritt (a) ein Bereich des dreidimensionalen Körpermodells entfernt wird, um einen Ort entsprechend dem Ort der Sonde (10) freizulegen.

6. Verfahren nach Anspruch 1,
**dadurch gekennzeichnet,**
daß das Anzeigen eines Bereichs des Datenbankkörpers (17) neben dem Datenbankort der Sonde (10) folgende Schritte aufweist:
a) Erzeugen eines Anzeigeformats unter Benutzung eines Strahlenabbildungsverfahrens auf zuvor aufgenommenen Bildern zum Darstellen eines Bereichs des Datenbankkörpers neben dem Datenbankort der Sonde; und
b) Anzeigen des Anzeigeformats.

7. Verfahren nach Anspruch 6,
**dadurch gekennzeichnet,**
daß das Strahlenabbildungsverfahren aus der Gruppe bestehend aus Röntgenstrahlung und Lichtstrahlung ausgewählt wird.

8. Verfahren nach Anspruch 6 oder 7,
**dadurch gekennzeichnet,**
daß das Anzeigeformat ein dreidimensionales Format ist.

9. Verfahren nach Anspruch 1, 2 oder 3,
**dadurch gekennzeichnet,**
daß die räumliche Orientierung der Sonde (10) zusammen mit ihrer räumlichen Position erhalten wird.

10. Verfahren nach Anspruch 1, 2 oder 3,
**dadurch gekennzeichnet,**
daß eine Darstellung der Sonde zusammen mit dem Bereich des Datenbankkörpers (17) neben dem Datenbankort der Sonde (10) angezeigt wird, und daß die relativen Orte der Dar stellung der Sonde (10) und des Datenbankkörpers (17) der räumlichen Position der Sonde (10) relativ zum anatomischen Körper (9) entsprechen.

11. Verfahren nach Anspruch 5,
**dadurch gekennzeichnet,**
daß eine Darstellung der Sonde (10) mit dem dreidimensionalen Körpermodell angezeigt wird, wobei der relative Ort der Darstellung der Sonde (10) zum dreidimensionalen Körpermodell der räumlichen Position der Sonde (10) relativ zum anatomischen Körper (9) entspricht.

12. Verfahren nach Anspruch 11,
**dadurch gekennzeichnet,**
daß die Darstellung der Sonde (10) genau der tatsächlichen Sonde entspricht, wobei die Darstellung der Sonde zusätzlich so orientiert ist, daß sie der Orientierung der Sonde bezüglich des anatomischen Körpers (9) entspricht, und wobei die Perspektive der Darstellung der Sonde (10) und des dreidimensionalen Körpermodells einander entsprechen.

13. Verfahren nach Anspruch 1,
gekennzeichnet durch den Schritt der Registrierung vor dem Erhalten der räumlichen Position, wobei die Registrierung folgende Schritte aufweist:
a) Positionieren der Sonde (10) neben einem bestimmten Merkmal des anatomischen Körpers (9);
b) Bestimmen einer räumlichen Position der Sonde (10);
c) Anzeigen eines Bereichs des Datenbankkörpers (17) mit einem Datenbankkörpermerkmal entsprechend dem bestimmten Merkmal;
d) Identifizieren des bestimmten Merkmals auf dem angezeigten Bereich; und
e) Registrieren der räumlichen Position der Sonde und des Ortes auf dem Datenbankkörper entsprechend der Position des bestimmten Merkmals;
wodurch ein Datenbankort bestimmt wird, der einer räumlichen Position der Sonde (10) entspricht.

14. Verfahren nach Anspruch 1,
gekennzeichnet durch den Schritt der Registrierung vor dem Erhalten der räumlichen Position, wobei die Registrierung folgende Schritte aufweist:
a) Markieren von Orten in dem Datenbankkörper (17), welche bestimmten Merkmalen des anatomischen Körpers (9) entsprechen;
b) Positionieren der Sonde (10) nahe einem bestimmten Merkmal des anatomischen Körpers (9);
c) Bestimmen der räumlichen Position der Sonde (10);
d) Registrieren der räumlichen Position der Sonde (10) und ihres entsprechenden Datenbankkörperorts;
wodurch ein Datenbankkörperort bestimmt wird, der mit einer räumlichen Position der Sonde (10) übereinstimmt.

15. Verfahren nach Anspruch 1,
gekennzeichnet durch den Schritt der Registrierung vor dem Erhalten der räumlichen Position, wobei die Registrierung folgende Schritte aufweist:
a) Markieren einer Position auf dem anatomischen Körper (9) eines bestimmten gescannten Bildes mit einem entsprechenden Ort im Datenbankkörper (17);
b) Positionieren der Sonde (10) nahe der markierten Position auf dem anatomischen Körper (9);
c) Bestimmen der räumlichen Position der Sonde (10);
d) Registrieren der räumlichen Position der Sonde (10) und ihres entsprechenden Datenbankkörperorts;
wodurch ein Datenbankkörperort bestimmt wird, der mit einer räumlichen Position der Sonde (10) übereinstimmt.

16. Verfahren nach Anspruch 13,
**dadurch gekennzeichnet,**
daß die Anzeige von Schritt (c) eine dreidimensionale Anzeige ist.

17. Verfahren nach Anspruch 13 oder 16.
**dadurch gekennzeichnet,**
daß mehr als drei Datenbankorte identifiziert werden und daß Fehler zwischen den entsprechenden Datenbankorten und den räumlichen Positionen minimalisiert werden, um die Genauigkeit des Registrierungsschritts zu verbessern.

18. Verfahren nach Anspruch 17,
**dadurch gekennzeichnet,**
daß die Fehler unter Benutzung einer Analyse der kleinsten Fehlerquadrate minimalisiert werden.

19. Verfahren nach Anspruch 13,
**dadurch gekennzeichnet,**
daß die Sonde (10) derart mit dem anatomischen Körper (9) verbunden ist, daß falls der anatomische Körper (9) nach der Registrierung verschoben wird, die Registrierung zwischen dem Datenbankkörper (17) und dem anatomischen Körper (9) aufrechterhalten wird.

20. System zum Sichtbarmachen innerer Bereiche eines anatomischen Körpers unter Benutzung eines Datenbankkörpers (17) zuvor aufgenommener Bilder des anatomischen Körpers (9), welches aufweist:
eine Sonde (10);
eine Datenbankspeichereinheit (12) mit den zuvor aufgenommenen Bildern des anatomischen Körpers (9);
einen räumlichen Determinator (20a, 20b, 36, 37, 39) zum Bestimmen der räumlichen Position der Sonde (10) relativ zum anatomischen Körper (9);
einen Computer (11), welcher die zuvor aufgenommenen Bilder benutzt, um eine Darstellung eines Bereichs des anatomischen Körpers (9) neben der räumlichen Position der Sonde (10) zu erzeugen;
eine Einrichtung (11) zum Abbilden und Registrieren der räumlichen Position der Sonde relativ zum anatomischen Körper (9) auf dem entsprechenden Datenbankort der Sonde (10) relativ zum Datenbankkörper (9);
eine Anzeigeeinheit (13) zum Anzeigen der Darstellung des anatomischen Körpers,
**dadurch gekennzeichnet,**
daß
die Sonde (10) und der anatomische Körper (9) jeweils eine Einrichtung (20a, 20b, 26, 27) aufweisen, welche es ermöglicht, daß der Determinator die räumliche Position der Sonde (10) relativ zum anatomischen Körper (9) derart bestimmt, daß die Sonde (10) und der anatomische Körper (9) unabhängig verschiebbar sind, und derart, daß die Registrierung zwischen dem Datenbankkörper (17) und dem anatomischen Körper (9) aufrechterhalten bleibt.

21. System nach Anspruch 20,
**dadurch gekennzeichnet,**
daß die erzeugten Darstellungen in einem dreidimensionalen Oberflächenformat angezeigt werden.

22. System nach Anspruch 20,
**dadurch gekennzeichnet,**
daß der Computer (11) derart gestaltet ist, daß er für den Ort in der Datenbankspeichereinheit entsprechend der räumlichen Position der Sonde (10) so initialisiert wird, daß er die Sonde neben einem bestimmten Merkmalspunkt des anatomischen Körpers (9) positioniert hat, daß eine räumliche Position der Sonde (10) bestimmt wird, daß ein Bereich des Datenbankkörpers (17) mit einem Datenbankkörpermerkmal entsprechend dem bestimmten Merkmal auf dem angezeigten Bereich angezeigt wird und daß die räumliche Position der Sonde (10) und der Ort auf dem Datenbankkörper (17) entsprechend der Position des bestimmten Merkmals registriert werden.

23. System nach Anspruch 22,
**dadurch gekennzeichnet,**
daß die erzeugten Bilder in einem dreidimensionalen Format während der Registrierung verschoben werden, und daß die bestimmten Merkmale auf dem dreidimensionalen Format identifiziert werden.

24. System nach Anspruch 20,
**dadurch gekennzeichnet,**
daß der räumliche Determinator (20a, 20b, 36, 37, 39) aufweist:
a) einen elektromagnetischen Emitter auf einem Referenzpunkt zum Übertragen eines Signales;
b) einen Sensor auf der Sonde (10) zum Empfangen des Signals; und
c) eine Einrichtung zum Vergleichen des übertragenen Signals mit dem empfangenden Signal zur Bestimmung der Position der Sonde (10).

25. System nach Anspruch 20,
**dadurch gekennzeichnet,**
daß der räumliche Determinator (20a, 20b, 36, 37, 39) aufweist:
a) erste, zweite, dritte und vierte Abschnitte;
b) einen Ständer;
c) ein erstes Gelenk zwischen dem ersten Abschnitt und der Sonde;
d) ein zweites Gelenk zwischen dem ersten und zweiten Abschnitt;
e) ein drittes Gelenk zwischen dem zweiten Abschnitt und dem Ständer;
f) ein viertes Gelenk zwischen dem Ständer und dem dritten Abschnitt;
g) ein fünftes Gelenk zwischen dem dritten Abschnitt und dem vierten Abschnitt;
h) ein sechstes Gelenk zwischen dem vierten Abschnitt und einem Referenzpunkt, dessen räumliche Position relativ zum anatomischen Körper bekannt ist;
e) Sensoren, welche an den Gelenken angeordnet sind; und
j) eine Einrichtung, die mit den Sensoren verbunden ist, zum Bestimmen der Position der Sonde relativ zum anatomischen Körper.

26. System nach Anspruch 20,
**dadurch gekennzeichnet,**
daß der räumliche Determinator (20, 20b, 36, 36, 39) die räumliche Orientierung der Sonde (10) sowie ihre räumliche Position bestimmt.

## Revendications

1. Procédé pour visualiser des régions internes d'un corps anatomique (9) en liaison avec une sonde (10), utilisant un corps base de données (17) d'images antérieurement saisies du corps anatomique (9), le procédé comprenant les étapes consistant à:
(a) obtenir une position spatiale de la sonde (10) par rapport au corps anatomique (9);
(b) déterminer un emplacement en base de données par rapport au corps base de données (17) correspondant à la position spatiale de la sonde (10) par rapport au corps anatomique (9);
(c) faire correspondre par application et registre à la position spatiale de la sonde (10) par rapport au corps anatomique (9) l'emplacement en base de données correspondant de la sonde (10) par rapport au corps base de données (17); et
(d) visualiser une région du corps base de données (17) se trouvant au voisinage de l'emplacement en base de données de la sonde (10), la région étant dérivée d'une pluralité d'images adjacentes du corps base de données (17),
caractérisé par
l'étape de détection des déplacements de chacun de ladite sonde (10) et dudit corps anatomique (9) pour permettre ladite obtention de ladite position spatiale de la sonde (10) par rapport audit corps anatomique (9) de manière à autoriser les déplacements indépendants de ladite sonde (10) et dudit corps anatomique (9) et de telle manière que le registre établi faisant correspondre le corps base de données(17) au corps anatomique (9) soit maintenu.

2. Procédé selon la revendication 1, caractérisé en ce que la visualisation d'une région du corps base de données (17) voisine de l'emplacement en base de données de la sonde (10) comprend les étapes consistant à:
(a) générer une image en coupe à partir des images antérieurement saisies et croisant une pluralité d'images adjacentes, représentant une région du corps base de données (17) voisine d'un emplacement en base de données de la sonde (10); et
(b) visualiser l'image en coupe.

3. Procédé selon la revendication 1, caractérisé en ce que la visualisation d'une région du corps base de données (17) voisine de l'emplacement en base de données de la sonde (10) comprend les étapes consistant à:
(a) générer un modèle de corps à trois dimensions à partir des images antérieurement saisies représentant une région du corps base de données voisine d'un emplacement en base de données de la sonde; et
(b) visualiser le modèle de corps à trois dimensions.

4. Procédé selon la revendication 1, caractérisé en ce que la visualisation d'une région du corps base de données voisine de l'emplacement en base de données de la sonde comprend les étapes consistant à:
(a) générer un modèle de corps à trois dimensions à partir d'images antérieurement saisies qui ont été prétraitées pour représenter des caractéristiques anatomiques et représentant une région du corps base de données voisine d'un emplacement en base de données de la sonde; et
(b) visualiser le modèle de corps à trois dimensions.

5. Procédé selon la revendication 4, caractérisé en ce que dans l'étape (a) une partie du modèle de corps à trois dimensions est enlevée afin de découvrir un emplacement correspondant à l'emplacement de la sonde (10).

6. Procédé selon la revendication 1, caractérisé en ce que la visualisation d'une région du corps base de données (17) voisine de l'emplacement en base de données de la sonde (10) comprend les étapes consistant à:
(a) générer une présentation de visualisation via l'utilisation d'un procédé à projection de rayons (ray-cast) sur des images antérieurement saisies représentant une région du corps base de données voisine d'un emplacement en base de données de la sonde; et
(b) visualiser la présentation de visualisation.

7. Procédé selon la revendication 6, caractérisé en ce que le procédé à projection de rayons est sélectionné dans le groupe formé par les rayons X et les rayons lumineux.

8. Procédé selon la revendication 6 ou 7, caractérisé en ce que la présentation de visualisation est une présentation à trois dimensions.

9. Procédé selon la revendication 1, 2 ou 3, caractérisé en ce que l'orientation spatiale de la sonde (10) est obtenue en même temps que sa position spatiale.

10. Procédé selon la revendication 1, 2 ou 3, caractérisé en ce qu'une représentation de la sonde est visualisée en même temps que la région du corps base de données (17) voisine de l'emplacement en base de données de la sonde (10) et les emplacements relatifs de la représentation de la sonde (10) et du corps base de données (17) correspondent à la position spatiale de la sonde (10) relativement au corps anatomique (9).

11. Procédé selon la revendication 5, caractérisé en ce qu'une représentation de la sonde (10) est visualisée en même temps que le modèle de corps à trois dimensions, l'emplacement de la représentation de la sonde (10) relativement au modèle de corps à trois dimensions correspondant à la position spatiale de la sonde (10) relativement au corps anatomique (9).

12. Procédé selon la revendication 11, caractérisé en ce que la représentation de la sonde (10) correspond étroitement à la sonde réelle, la représentation de la sonde étant en outre orientée de manière correspondant à l'orientation de la sonde par rapport au corps anatomique (9), et la perspective de la représentation de la sonde (10) correspondant à celle du modèle de corps à trois dimensions.

13. Procédé selon la revendication 1, caractérisé par le fait qu'il comprend en outre une étape d'établissement de registre avant l'obtention de la position spatiale, l'établissement de registre comprenant les étapes consistant à:
(a) positionner la sonde (10) à côté d'une caractéristique particulière du corps anatomique (9);
(b) déterminer une position spatiale de la sonde (10);
(c) visualiser une région du corps base de données (17) ayant une caractéristique de corps base de données correspondant à la caractéristique particulière;
(d) identifier la caractéristique particulière sur la région visualisée; et
(e) enregistrer la position spatiale de la sonde et l'emplacement sur le corps base de données qui correspondent à la position de la caractéristique particulière;
ce qui fait qu'est déterminée une correspondance entre un emplacement en base de données et une position spatiale de la sonde (10).

14. Procédé selon la revendication 1, caractérisé par le fait qu'il comprend en outre une étape d'établissement de registre avant l'obtention de la position spatiale, l'établissement de registre comprenant les étapes consistant à:
(a) marquer des emplacements dans le corps base de données (17) qui correspondent à des caractéristiques particulières du corps anatomique (9);
(b) positionner la sonde (10) à côté d'une caractéristique particulière du corps anatomique (9);
(c) déterminer la position spatiale de la sonde (10);
(d) enregistrer la position spatiale de la sonde (10) et son emplacement correspondant sur le corps base de données;
ce qui fait qu'est déterminée une correspondance entre un emplacement sur le corps base de données et une position spatiale de la sonde (10).

15. Procédé selon la revendication 1, caractérisé par le fait qu'il comprend en outre une étape d'établissement de registre avant l'obtention de la position spatiale, l'établissement de registre comprenant les étapes consistant à:
(a) marquer une position sur le corps anatomique (9) d'une image lue particulière contenant un emplacement correspondant dans le corps base de données (17);
(b) positionner la sonde (10) à côté de la position marquée sur le corps anatomique (9);
(c) déterminer la position spatiale de la sonde (10);
(d) enregistrer la position spatiale de la sonde (10) et son emplacement correspondant sur le corps base de données; ce qui fait qu'est déterminée une correspondance entre un emplacement sur le corps base de données et une position spatiale de la sonde (10).

16. Procédé selon la revendication 13, caractérisé en ce que la visualisation de l'étape (c) est une visualisation en trois dimensions.

17. Procédé selon la revendication 13 ou 16, caractérisé en ce que plus de trois emplacements en base de données sont identifiés et en ce que les erreurs intervenant entre les emplacements en base de données et les positions spatiales qui correspondent sont minimisées afin d'améliorer la précision de l'étape d'établissement de registre.

18. Procédé selon la revendication 17, caractérisé en ce que les erreurs sont minimisées en utilisant au moins l'analyse par carrés moyens.

19. Procédé selon la revendication 13, caractérisé en ce que la sonde (10) est liée au corps anatomique (9) de sorte que si, après établissement de registre, le corps anatomique (9) est déplacé, le registre établi faisant correspondre le corps base de données (17) au corps anatomique (9) est maintenu.

20. Système pour visualiser des régions internes d'un corps anatomique en utilisant un corps base de données (17) d'images antérieurement saisies du corps anatomique (9), le système comprenant:
une sonde (10);
une unité de mémorisation de base de données (12) contenant les images antérieurement saisies du corps anatomique (9);
un moyen de détermination spatiale (20a, 20b, 36, 37, 39) pour déterminer la position spatiale de la sonde (10) relativement au corps anatomique (9);
un ordinateur (11) utilisant les images antérieurement saisies pour générer une représentation d'une région du corps anatomique (9) voisine de la position spatiale de la sonde (10);
un moyen (11) pour faire correspondre par application et registre à ladite position spatiale de la sonde par rapport au corps anatomique (9) l'emplacement en base de données correspondant de la sonde (10) par rapport au corps base de données (9); et
une unité de visualisation (13) pour visualiser la représentation du corps anatomique,
caractérisé en ce que
ladite sonde (10) et ledit corps anatomique (9) ont chacun des moyens (20a, 20b, 26, 27) pour permettre audit moyen de détermination de déterminer la position spatiale de la sonde (10) par rapport au corps anatomique (9) de manière à autoriser les déplacements indépendants de la sonde (10) et du corps anatomique (9) de telle manière que le registre établi faisant correspondre le corps base de données (17) au corps anatomique (9) soit maintenu.

21. Système selon la revendication 20, caractérisé en ce que les représentations générées sont visualisées dans une présentation de surface à trois dimensions.

22. Système selon la revendication 20, caractérisé en ce que l'ordinateur (11) est adapté à être initialisé pour l'emplacement dans l'unité de mémorisation de base de données correspondant à la position spatiale de la sonde (10) en positionnant la sonde à côté d'un point caractéristique particulier du corps anatomique (9), en déterminant une position spatiale de la sonde (10), en visualisant une région du corps base de données (17) ayant une caractéristique de corps base de données qui correspond à la caractéristique particulière sur la région visualisée, et en enregistrant la position spatiale de la sonde (10) et l'emplacement sur le corps base de données (17) correspondant à la position de la caractéristique particulière.

23. Système selon la revendication 22, caractérisé en ce que les images générées sont déplacées en présentation à trois dimensions pendant l'établissement de registre, et les caractéristiques particulières sont identifiées sur la présentation à trois dimensions.

24. Système selon la revendication 20, caractérisé en ce que le moyen de détermination spatiale (20a, 20b, 36, 37, 39) comprend:
(a) un émetteur électromagnétique sur un point de référence pour transmettre un signal;
(b) un capteur sur la sonde (10) pour recevoir le signal; et
(c) un moyen pour comparer le signal transmis avec le signal reçu afin de déterminer la position de la sonde

25. Système selon la revendication 20, caractérisé en ce que le moyen de détermination spatiale (20a, 20b, 36, 37, 39) comprend:
(a) une première, une seconde, une troisième et une quatrième sections;
(b) un pied;
(c) une première articulation entre la première section et la sonde;
(d) une seconde articulation entre la première et la seconde section;
(e) une troisième articulation entre la seconde section et le pied;
(f) une quatrième articulation entre le pied et la troisième section;
(g) une cinquième articulation entre la troisième section et la quatrième section;
(h) une sixième articulation entre la quatrième section et un point de référence dont la position spatiale relativement au corps anatomique est connue;
(i) des détecteurs intervenant au niveau de chacune des articulations; et
(j) un moyen connecté aux détecteurs pour déterminer la position de la sonde relativement au corps anatomique.

26. Système selon la revendication 20, caractérisé en ce que le moyen de détermination spatiale (20a, 20b, 36, 37, 39) détermine l'orientation spatiale de la sonde (10) aussi bien que la position spatiale de cette dernière.
